# EUROPEAN PATENT SPECIFICATION

(11) **EP 4 649 958 B1**
(45) Date of publication and mention of the grant of the patent: **02.12.2026**
(21) Application number: 24741164.8
(22) Date of filing: 05.01.2024
(51) Int. Cl.: A61K 39/295, A61K 39/245, A61K 39/12, A61K 39/23, A61K 39/39, A61P 31/14, A61P 31/22, A61P 31/20, C07K 14/005, C12N 15/40, C12N 15/38, C12N 15/35

(54) **TRIPLE MRNA VACCINE FOR PREVENTING FELINE RHINOTRACHEITIS, FELINE CALICIVIRUS DISEASES, AND FELINE PANLEUKOPENIA, AND PREPARATION METHOD THEREFOR**
DREIFACH-MRNA-IMPFSTOFF ZUR VORBEUGUNG VON RHINOTRACHEITIS, KATZENCALICIVIRUSERKRANKUNGEN UND KATZENPANLEUKOPENIE SOWIE HERSTELLUNGSVERFAHREN DAFÜR
TRIPLE VACCIN À ARNM POUR PRÉVENTION DE LA RHINOTRACHÉITE FÉLINE, DE CALICIVIROSES FÉLINES ET DE LA PANLEUCOPÉNIE FÉLINE, ET SON PROCÉDÉ DE PRÉPARATION

(30) Priority: 10.01.2023 CN 202310037630
(43) Date of publication of application: 19.11.2025
(73) Proprietor: Zhejiang University, Hangzhou, Zhejiang 310058 (CN)
(72) Inventor: TANG, Jianbin, Hangzhou, Zhejiang 310058 (CN); YANG, Yongle, Hangzhou, Zhejiang 310058 (CN); HUANG, Yaowei, Hangzhou, Zhejiang 310058 (CN)
(74) Representative: ZBM Patents - Zea, Barlocci & Markvardsen
(86) International application number: PCT/CN2024/070795
(87) International publication number: WO 2024/149165

(56) References cited:
- CN-A- 110 714 015
- CN-A- 111 632 137
- CN-A- 114 015 702
- CN-A- 115 969 967
- CN-A- 116 042 679
- CN-A- 116 121 282
- CN-A- 116 218 889
- JP-A- 2014 214 088
- SATO E ET AL: "Further development of a recombinant feline herpesvirus type 1 expressing the Gag protein of feline immunodeficiency virus", ARCHIVES OF VIROLOGY, SPRINGER VIENNA, AUSTRIA, vol. 146, no. 2, 1 March 2001 (2001-03-01), pages 379 - 387, XP037139931, ISSN: 0304-8608, DOI: 10.1007/S007050170182
- TENCHOV RUMIANA, BIRD ROBERT, CURTZE ALLISON E., ZHOU QIONGQIONG: "Lipid Nanoparticles─From Liposomes to mRNA Vaccine Delivery, a Landscape of Research Diversity and Advancement", ACS NANO, AMERICAN CHEMICAL SOCIETY, US, vol. 15, no. 11, 23 November 2021 (2021-11-23), US , pages 16982 - 17015, XP093009618, ISSN: 1936-0851, DOI: 10.1021/acsnano.1c04996
- LE TING, SUN CHAO, CHANG JITAO, ZHANG GUIJIE, YIN XIN: "mRNA Vaccine Development for Emerging Animal and Zoonotic Diseases", VIRUSES, MDPI, CH, vol. 14, no. 2, 15 February 2022 (2022-02-15), CH , pages 401, XP093192289, ISSN: 1999-4915, DOI: 10.3390/v14020401

## Description

### TECHNICAL FIELD

The present invention relates to the field of nucleic acid vaccines, and in particular, to a triple mRNA vaccine for preventing feline rhinotracheitis, feline caliciviral disease, and feline panleukopenia, and a preparation method thereof.

### BACKGROUND

Feline viral rhinotracheitis (FVR), also known as feline infectious rhinotracheitis, is an acute and highly contagious disease in cats caused by feline herpesvirus (FHV) infection. The typical clinical symptoms of FHV infection include elevated body temperature, sneezing, coughing, keratoconjunctivitis, serous or purulent nasal and ocular secretions, and periocular skin ulcers. It can occur in both kittens and adult cats, mainly infecting those aged 2-4 months with a morbidity rate of up to 100%. While it does not cause death in adult cats, the mortality rate in kittens can reach 50%, making it one of the most severe known feline respiratory diseases. The gB protein of FHV is an envelope glycoprotein composed of 949 amino acids encoded by the UL27 gene. It is an important component of the FHV envelope surface and the most dominant immunogenic protein, playing a decisive role in virus invasion of cells, virus replication, cell fusion, and intercellular transmission. The gD protein of FHV, a major component of the viral envelope, is present on the membrane of infected cells. It has high conservativeness and antigenicity, can specifically bind to molecules on the cell surface, induce the body to produce cellular and humoral immunity, and plays an important role in viral penetration into susceptible cells. The protein is one of the main target cells of the host's cellular and humoral immune responses. Both gB and gD proteins are extremely conserved immunogenic proteins, serve as target proteins commonly selected for studying herpesvirus subunit vaccines, and have good immunogenicity.

Feline caliciviral disease (FCVD) is a disease mainly characterized by upper respiratory tract infections caused by feline calicivirus (FCV). The disease mainly affects kittens aged 7-84 days and often occurs at 56-84 days old. Cats under 1 year old are the most susceptible. The younger the cat is in days, the higher the mortality rate after infection. The incubation period is 2-3 days, and the natural course of the disease is 7-10 days. Its main clinical symptoms include oral ulcers, pneumonia, chronic gastritis, arthritis, and lameness, etc. The VP1 protein of FCV is a structural protein that plays a key role in virion assembly, antigenic determination, and virus-host cell interactions. VP1 contains most neutralizing antibody epitopes and is a key research target.

Feline panleukopenia (FP) is an acute and highly contagious disease in cats caused by feline parvovirus (FPV). The typical clinical symptoms of FPV infection include biphasic fever, vomiting, diarrhea, dehydration, circulatory disturbance, severe leukopenia, and hemorrhagic enteritis. It mainly infects kittens under 1 year old. Generally, the lethality rate for cats under 1 year old is 50%-60%, and the mortality rate for kittens under 5 months old is as high as 80%-90%. The VP2 protein of FPV accounts for about 90% of all structural proteins, while the VP1 protein accounts for 10%. Therefore, the VP2 protein is the main antigenic protein encoded by FPV, playing an important role in immune responses, receptor recognition, and the process of infecting tissues. The VP2 protein has multiple antigen recognition sites, which can stimulate the body to produce neutralizing antibodies and play a role in self-protection.

Research and development of mRNA vaccines for preventing feline rhinotracheitis, feline caliciviral disease, and feline panleukopenia are of positive significance for the prevention and control of major feline diseases. Compared with traditional vaccines, mRNA vaccines, as a novel form of vaccine, have the advantages of simple production processes, fast development speeds, no need for cell culture, and low costs. Compared with DNA vaccines, mRNA vaccines do not need to enter the nucleus, eliminating the risk of integration into the host genome, and their half-life can be adjusted through modification. mRNA vaccines can provide a comprehensive stimulation of adaptive and innate immunity, namely in-situ antigen expression and danger signal transmission. They can induce a "balanced" immune response, including humoral and cellular effectors as well as immunological memory. Clinically, co-infections of the above three feline diseases are relatively common, often complicated by secondary bacterial infections, leading to more complex symptoms. In related technologies, a triple mRNA vaccine targeting the above three viruses is still lacking. Once infection occurs, symptomatic treatment or antibiotic administration is commonly used for therapy, but the therapeutic effect and specificity are poor, and drug resistance and toxic side effects are prevalent.

SATO E ET AL: "Further development of a recombinant feline herpesvirus type 1 expressing the Gag protein of feline immunodeficiency virus", ARCHIVES OF VIROLOGY, SPRINGER VIENNA, AUSTRIA, vol. 146, no. 2, 1 March 2001 discloses a recombinant feline herpesvirus type 1 expressing the Gag protein of FH-1 used for inducing anti-FIV Gag. It also discloses that trivalent vaccine are commonly used for prophylaxis of infections of feline panleukopenia virus, feline calicivirius and feline herpesvirus. The most widely used trivalent vaccine in Japan consists of inactivated FPLV, together with attenuated FCV and FHV-1.

### SUMMARY

The present invention provides a triple mRNA vaccine for preventing feline rhinotracheitis, feline caliciviral disease, and feline panleukopenia, and a preparation method thereof.

The triple mRNA vaccine for preventing feline rhinotracheitis, feline caliciviral disease, and feline panleukopenia provided by the present invention can offer effective immunoprotection against the three major epidemic infectious diseases in cats. Moreover, the triple mRNA vaccine provided by the present invention only requires a single immunization to achieve a goal of simultaneously preventing and controlling the above three feline epidemic infectious diseases, which is of positive significance for prevention and control of feline epidemic diseases.

Technical solutions of the present invention:
A triple mRNA vaccine for preventing feline rhinotracheitis, feline caliciviral disease, and feline panleukopenia, including an mRNA expressing an antigenic protein, where the mRNA expressing the antigenic protein includes an mRNA expressing a feline herpesvirus gB protein, an mRNA expressing a feline herpesvirus gD protein, an mRNA expressing a feline calicivirus VP1 protein, and an mRNA expressing a feline parvovirus VP2 protein.

A gene encoding the feline herpesvirus gB protein has a deletion of a nucleotide sequence of positions 2440-2847 at a 3' end, a gene encoding the feline herpesvirus gD protein has a deletion of a nucleotide sequence of positions 988-1125 at a 3' end, and a gene encoding the feline calicivirus VP1 protein has a deletion of a nucleotide sequence of positions 4-372 at a 5' end.

The gene encoding the feline herpesvirus gB protein is a codon-optimized gene with a nucleotide sequence as shown in SEQ ID NO.1; the gene encoding the feline herpesvirus gD protein is a codon-optimized gene with a nucleotide sequence as shown in SEQ ID NO.2; the gene encoding the feline calicivirus VP1 protein is a codon-optimized gene with a nucleotide sequence as shown in SEQ ID NO.3; and a gene encoding the feline parvovirus VP2 protein is a codon-optimized gene with a nucleotide sequence as shown in SEQ ID NO.4.

Preferably, an mRNA sequence expressing the feline herpesvirus gB protein is as shown in SEQ ID NO.5 or SEQ ID NO.9, an mRNA sequence expressing the feline herpesvirus gD protein is as shown in SEQ ID NO.6 or SEQ ID NO.10, an mRNA sequence expressing the feline calicivirus VP1 protein is as shown in SEQ ID NO.7 or SEQ ID NO.11, and an mRNA sequence expressing the feline parvovirus VP2 protein is as shown in SEQ ID NO.8 or SEQ ID NO.12.

A mass ratio of the mRNA expressing the feline herpesvirus gB protein, the mRNA expressing the feline herpesvirus gD protein, the mRNA expressing the feline calicivirus VP1 protein, and the mRNA expressing the feline parvovirus VP2 protein is 2:2:3:3.

The triple mRNA vaccine further includes an adjuvant, where the adjuvant includes a lipid nanoparticle adjuvant, an oil-in-water adjuvant, a polymer-and-water-based adjuvant, a water-in-oil adjuvant, an aluminum hydroxide adjuvant, or a vitamin E adjuvant. The adjuvant is a cationic lipid, distearoylphosphatidylcholine, a polyethylene glycol lipid, and cholesterol at a molar ratio of 50:10:38.5:1.5.

Specifically, the triple mRNA vaccine is in a dosage form for intramuscular, intradermal, or subcutaneous administration.

The present invention further provides a method for preparing the triple mRNA vaccine, including the following steps:
(1) constructing a synthetic plasmid for an mRNA expressing an antigenic protein, and performing linearization treatment with a restriction endonuclease to obtain a linearized synthetic plasmid for the mRNA expressing the antigenic protein;
(2) performing an in vitro transcription reaction on the linearized synthetic plasmid for the mRNA expressing the antigenic protein using a T7 promoter sequence, and purifying to obtain the mRNA expressing the antigenic protein;
(3) performing a Cap capping modification reaction on the mRNA expressing the antigenic protein obtained in the step (2) by an enzymatic method using a vaccinia virus capping enzyme and 2'-O-methyltransferase, and purifying to obtain a cap-modified mRNA expressing the antigenic protein; and
(4) mixing the cap-modified mRNA expressing the antigenic protein obtained in the step (3) with a cationic lipid, distearoylphosphatidylcholine, cholesterol, and a polyethylene glycol lipid to obtain the triple mRNA vaccine.

The synthetic plasmid for the mRNA expressing the antigenic protein includes a T7 promoter sequence, a 5'UTR region, an mRNA sequences encoding a feline herpesvirus gB protein or a feline herpesvirus gD protein or a feline calicivirus VP1 protein or a feline parvovirus VP2 protein, a 3'UTR region, and a 3'-terminal poly(A) tail.

The synthetic plasmid for the mRNA can be constructed in any cloning vector, with a restriction endonuclease site retained at the end of the poly(A) tail for plasmid linearization preparation. Preferably, the restriction endonuclease site is *BspQ* I, *Bsa* I, or *Mlu* I.

Currently, mRNA vaccines mainly exist in two sequence structures: traditional non-replicating mRNA sequences and self-amplifying (replicating) mRNA vaccine sequences. Non-replicating mRNA vaccines have a simple structure and cannot self-replicate in vivo. They require mature optimization processes to induce immune responses at low doses. Self-amplifying mRNA sequences contain replicase genes, which can amplify mRNAs in cells, thereby producing more antigens with lower mRNA doses.

The mRNA expressing the feline herpesvirus gB protein, the mRNA expressing the feline herpesvirus gD protein, the mRNA expressing the feline calicivirus VP1 protein, and the mRNA expressing the feline parvovirus VP2 protein further include coding regions of alphavirus non-structural protein genes. The coding regions of the alphavirus non-structural protein genes are coding regions of non-structural protein 1-4 genes. Preferably, the alphavirus is Venezuelan equine encephalitis (VEE) virus. An nsp 1-4 region of a VEE replicase gene contains a VEE virus replicase gene sequence, and an expressed VEE virus replicase can synthesize mRNAs in vivo, serving the function of replicating mRNAs.

The present invention further provides use of the triple mRNA vaccine in preparation of a medicament for simultaneously preventing feline rhinotracheitis, feline caliciviral disease, and feline panleukopenia.

Animals immunized with an LNP-mRNA vaccine have specific antibodies against feline herpesvirus, feline calicivirus, and feline parvovirus in vivo, demonstrating the effect of preventing infections by feline herpesvirus, feline calicivirus, and feline parvovirus.

Beneficial effects of the present invention:
The present invention prepares a triple mRNA vaccine for feline rhinotracheitis, feline caliciviral disease, and feline panleukopenia. The preparation includes combining an mRNA expressing a feline herpesvirus gB protein, an mRNA expressing a feline herpesvirus gD protein, an mRNA expressing a feline calicivirus VP1 protein, and an mRNA expressing a feline parvovirus VP2 protein with a liposome encapsulation protocol to prepare the mRNA vaccine. In addition, the vaccine is applied for immunization against feline herpesvirus, feline calicivirus, and feline parvovirus. The method holds application value in the research and vaccine development of major epidemic infectious diseases in cats, and can strongly promote the translational application of the vaccine.

### BRIEF DESCRIPTION OF THE DRAWINGS

FIG. 1 is a schematic diagram of synthetic plasmids for non-replicating mRNAs expressing antigenic proteins of a triple mRNA vaccine for feline rhinotracheitis, feline caliciviral disease, and feline panleukopenia (including T7-FHV-gB and T7-FHV-gD).
FIG. 2 is a schematic diagram of synthetic plasmids for non-replicating mRNAs expressing antigenic proteins of a triple mRNA vaccine for feline rhinotracheitis, feline caliciviral disease, and feline panleukopenia (including T7-FCV-VP1 and T7-FPV-VP2).
FIG. 3 is a schematic diagram of synthetic plasmids for replicating mRNAs expressing antigenic proteins of a triple mRNA vaccine for feline rhinotracheitis, feline caliciviral disease, and feline panleukopenia (including VEE-FHV-gB and VEE-FHV-gD).
FIG. 4 is a schematic diagram of synthetic plasmids for replicating mRNAs expressing antigenic proteins of a triple mRNA vaccine for feline rhinotracheitis, feline caliciviral disease, and feline panleukopenia (including VEE-FCV-VP1 and VEE-FPV-VP2).
FIG. 5 is an image showing agarose gel electrophoresis verification results for linearization by restriction digestion of synthetic plasmids for non-replicating mRNAs expressing antigenic proteins of a triple mRNA vaccine, where M: DNA markers, 1: synthetic plasmids for non-replicating mRNAs expressing antigenic proteins, and 2: linearization by restriction digestion of synthetic plasmids for non-replicating mRNAs expressing antigenic proteins.
FIG. 6 is an image showing agarose gel electrophoresis verification results for linearization by restriction digestion of synthetic plasmids for replicating mRNAs expressing antigenic proteins of a triple mRNA vaccine, where M: DNA markers, 1: synthetic plasmids for replicating mRNAs expressing antigenic proteins, and 2: linearization by restriction digestion of synthetic plasmids for replicating mRNAs expressing antigenic proteins.
FIG. 7 is an image showing agarose gel electrophoresis verification results for cap-modified non-replicating mRNAs expressing antigenic proteins (including cap-gB-mRNA, cap-gD-mRNA, cap-VP1-mRNA, and cap-VP2-mRNA), where M: DNA markers, 1: synthetic plasmids for non-replicating mRNAs expressing antigenic proteins, and 2: cap-modified non-replicating mRNAs expressing antigenic proteins.
FIG. 8 is an image showing agarose gel electrophoresis verification results for cap-modified replicating mRNAs expressing antigenic proteins (including cap-VEE-gB-mRNA, cap-VEE-gD-mRNA, cap-VEE-VP1-mRNA, and cap-VEE-VP2-mRNA).
FIG. 9 is an image showing indirect immunofluorescence assay results for expression of a feline herpesvirus gB protein, a feline herpesvirus gD protein, a feline calicivirus VP1 protein, and a feline parvovirus VP2 protein after transfection of cap-modified non-replicating mRNAs expressing antigenic proteins into cells.
FIG. 10 is an image showing indirect immunofluorescence assay results for expression of a feline herpesvirus gB protein, a feline herpesvirus gD protein, a feline calicivirus VP1 protein, and a feline parvovirus VP2 protein after transfection of cap-modified replicating mRNAs expressing antigenic proteins into cells.
FIG. 11 is a diagram showing results of enzyme-linked immunosorbent assay for detecting a specific antibody level against feline calicivirus in serum of cats immunized with a triple mRNA vaccine for feline rhinotracheitis, feline caliciviral disease, and feline panleukopenia.
FIG. 12 is a diagram showing results of enzyme-linked immunosorbent assay for detecting a specific antibody level against feline parvovirus in serum of cats immunized with a triple mRNA vaccine for feline rhinotracheitis, feline caliciviral disease, and feline panleukopenia.
FIG. 13 is a diagram showing results of enzyme-linked immunosorbent assay for detecting a specific antibody level against feline herpesvirus in serum of cats immunized with a triple mRNA vaccine for feline rhinotracheitis, feline caliciviral disease, and feline panleukopenia.

### DESCRIPTION OF THE EMBODIMENTS

The technical solutions of the present invention will be further described in detail below through specific embodiments, but the present invention is not therefore limited to the scope of the described examples.

The triple mRNA vaccine developed for feline rhinotracheitis, feline caliciviral disease, and feline panleukopenia in the present invention mainly adopts two methods: (1) preparing cap-modified mRNAs and replicating mRNAs expressing antigenic proteins of feline herpesvirus, feline calicivirus, and feline parvovirus; and (2) preparing a novel LNP-mRNA vaccine.

### Example 1 Construction of Synthetic Plasmids for mRNAs Expressing Antigenic Proteins in a Triple mRNA Vaccine for Feline Rhinotracheitis, Feline Caliciviral Disease, and Feline Panleukopenia

The gene sequences of the gB and gD proteins of feline herpesvirus (FHV), the gene sequence of the VP1 protein of feline calicivirus (FCV), and the gene sequence of the VP2 protein of feline parvovirus (FPV) are preserved in the College of Animal Sciences, Zhejiang University (reference sequences: FHV FJ478159, FCV M86379, and FPV MT614366).

According to transmembrane sequence analysis of proteins and protein cleavage analysis, the gene of the feline herpesvirus gB protein was designed to have a deletion of the nucleotide sequence of positions 2440-2847 at the 3' end, the gene of the feline herpesvirus gD protein was designed to have a deletion of the nucleotide sequence of positions 988-1125 at the 3' end, and the gene of the feline calicivirus VP1 protein was designed to have a deletion of the nucleotide sequence of positions 4-372 at the 5' end. In addition, the above modified gene sequences of the antigenic proteins and the gene sequence of the feline parvovirus VP2 protein were codon-optimized for mammalian expression, and the sequences are as shown in SEQ ID NO.1-4.

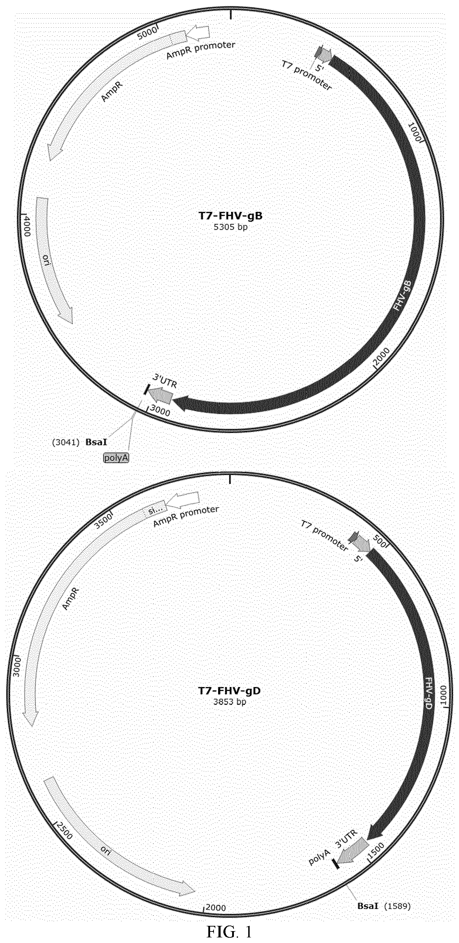

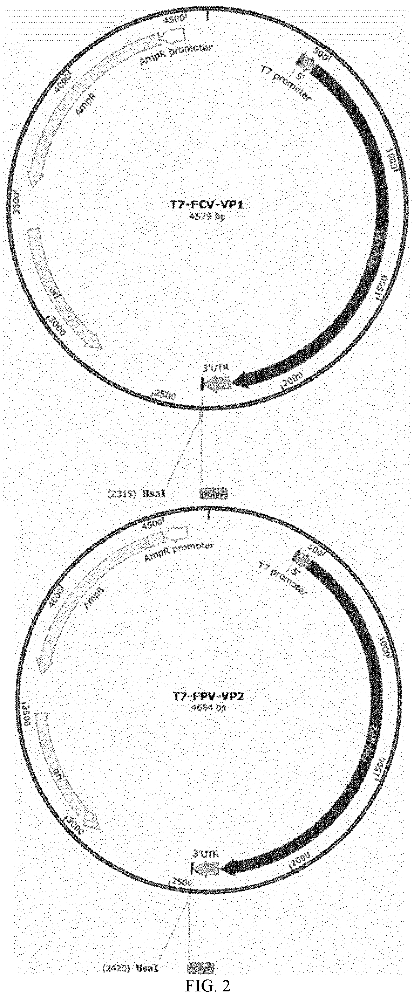

As shown in FIGS. 1 and 2, construction was performed in the pUC cloning vector in the order of the T7 promoter sequence, the 5' UTR region, the modified codon-optimized gene sequence expressing the antigenic protein, the 3' UTR region, and the 3' terminal poly(A) tail. Synthetic plasmids for mRNAs expressing antigenic proteins were obtained by gene synthesis, including T7-FHV-gB, T7-FHV-gD, T7-FCV-VP1, and T7-FPV-VP2. There was a *Bsa* I restriction endonuclease site at the end of the poly(A) tail for plasmid linearization preparation.

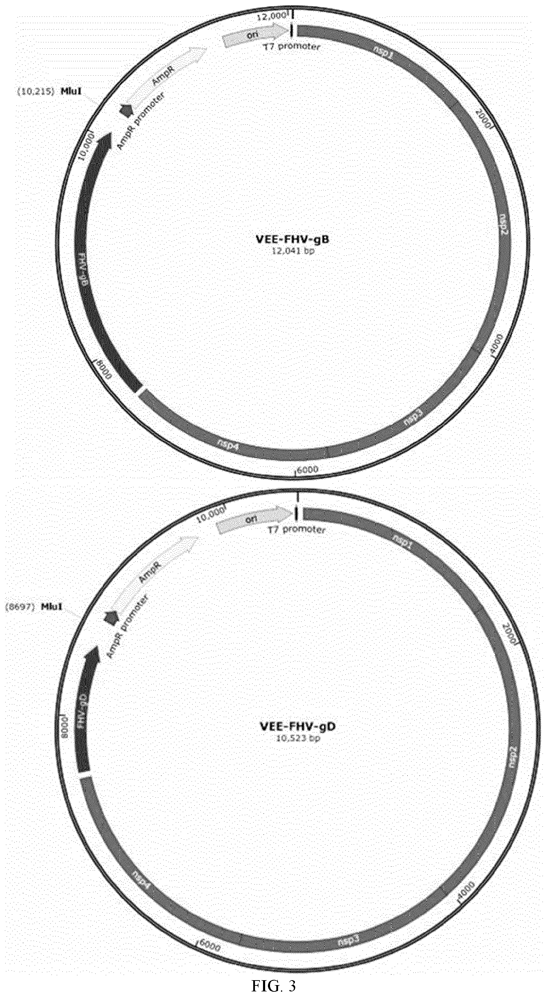

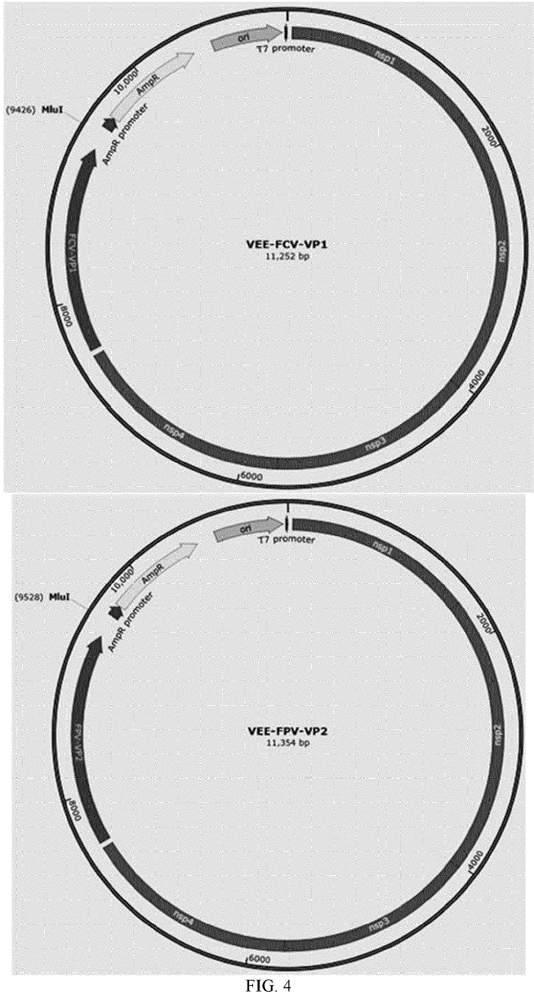

As shown in FIGS. 3 and 4, construction was performed in the VEE cloning vector in the order of the T7 promoter sequence, the 5' UTR region, the VEE replicase gene nsp 1-4 region, the modified codon-optimized gene sequence expressing the antigenic protein, the 3' UTR region, and the 3' terminal poly(A) tail. Replicating mRNA plasmids were obtained through construction, including VEE-FHV-gB, VEE-FHV-gD, VEE-FCV-VP1, and VEE-FPV-VP2V. There was an *Mlu* I restriction endonuclease site at the end of the poly(A) tail for plasmid linearization preparation.

The plasmids T7-FHV-gB, T7-FHV-gD, T7-FCV-VP1, T7-FPV-VP2, as well as VEE-FHV-gB, VEE-FHV-gD, VEE-FCV-VP1, and VEE-FPV-VP2 were preserved in *Escherichia coli* T1 competent strains. Single clones were randomly picked, separately inoculated into 200 mL of LB liquid medium containing ampicillin resistance, and cultured in a shaker at 37°C. The synthetic plasmids for mRNAs expressing antigenic proteins obtained by cloning were extracted according to the instructions of the Omega Plasmid DNA Maxi Kit. After determining the concentration, they were stored for subsequent use.

### Example 2 In Vitro Transcription for Preparation of mRNAs Expressing Antigenic Proteins

As shown in FIGS. 1 and 2, the synthetic plasmids for mRNAs expressing a feline herpesvirus gB protein, a feline herpesvirus gD protein, a feline calicivirus VP1 protein, and a feline parvovirus VP2 protein (T7-FHV-gB, T7-FHV-gD, T7-FCV-VP1, and T7-FPV-VP2) retained a *Bsa* I restriction endonuclease site at the end of the poly(A) tail, and the vector plasmids were subjected to a linearization reaction by restriction digestion using a restriction endonuclease *Bsa* I. As shown in FIGS. 3 and 4, the synthetic plasmids for replicating mRNAs expressing a feline herpesvirus gB protein, a feline herpesvirus gD protein, a feline calicivirus VP1 protein, and a feline parvovirus VP2 protein (VEE-FHV-gB, VEE-FHV-gD, VEE-FCV-VP1, and VEE-FPV-VP2) retained an *Mlu* I restriction endonuclease site at the end of the poly(A) tail, and the vector plasmids were subjected to a linearization reaction by restriction digestion using a restriction endonuclease *Mlu* I. The two linearization reactions by restriction digestion were performed according to the following steps:
(1) The synthetic plasmids for mRNAs and the synthetic plasmids for replicating mRNAs constructed according to Example 1 were subjected to linearization by restriction digestion with the endonuclease *Bsa* I or the endonuclease *Mlu* I.
a) First, 25 µg of T7-FHV-gB, T7-FHV-gD, T7-FCV-VP1, or T7-FPV-VP2 plasmids were taken and digested with *Bsa* I at 37°C for 2 h. The restriction digestion reaction system was prepared as follows:

| | |
|---|---|
| mRNA synthetic plasmid DNA | 25 µg, |
| 10× buffer | 10 µL, |
| Restriction endonuclease *Bsa* I | 5 µL, |
| Nuclease-free water | adjust the volume to 100 µL; |

25 µg of VEE-FHV-gB, VEE-FHV-gD, VEE-FCV-VP1, or VEE-FPV-VP2 plasmids were taken and digested with *Mlu* I at 37°C for 2 h. The restriction digestion reaction system was prepared as follows:

| | |
|---|---|
| Replicating mRNA synthetic plasmid DNA | 25 µg, |
| 10× buffer | 10 µL, |
| Restriction endonuclease *Mlu* I | 5 µL, |
| Nuclease-free water | adjust the volume to 100 µL; |

b) After restriction digestion, 5 µL of 10% SDS solution was added to achieve a final SDS concentration of 0.5%;
c) 0.5 µL of 20 mg/µL proteinase K was added to achieve a final proteinase concentration of 50-100 µg/mL;
d) The mixture was incubated at 37°C for 1 h, then placed on ice. 200 µL of nuclease-free water and 300 µL of phenol/CHCl3/IAA mixture were added, vortexed, and then allowed to stand for 5 min;
e) The mixture was centrifuged at a rotational speed of 12,000 rpm for 10 min at room temperature. The supernatant was aspirated, added with 750 µL of anhydrous ethanol, and allowed to stand in a refrigerator at -20°C for 30 min;
f) The mixture was centrifuged at a rotational speed of 12,000 rpm for 15 min at 4°C, and the supernatant was discarded;
g) 1 mL of 75% ethanol was added, and the mixture was centrifuged at a rotational speed of 12,000 rpm for 5 min at 4°C. This step was repeated twice, and the supernatant was discarded. The centrifuge tube was air-dried;
h) The DNA was dissolved by adding 20 µL of nuclease-free water. 1 µL was taken and diluted 10-fold to determine the concentration of the linearized DNA, and the DNA was stored at -20°C; and
i) The linearization by restriction digestion of the synthetic plasmids for the mRNAs expressing the antigenic proteins was verified by agarose gel electrophoresis.

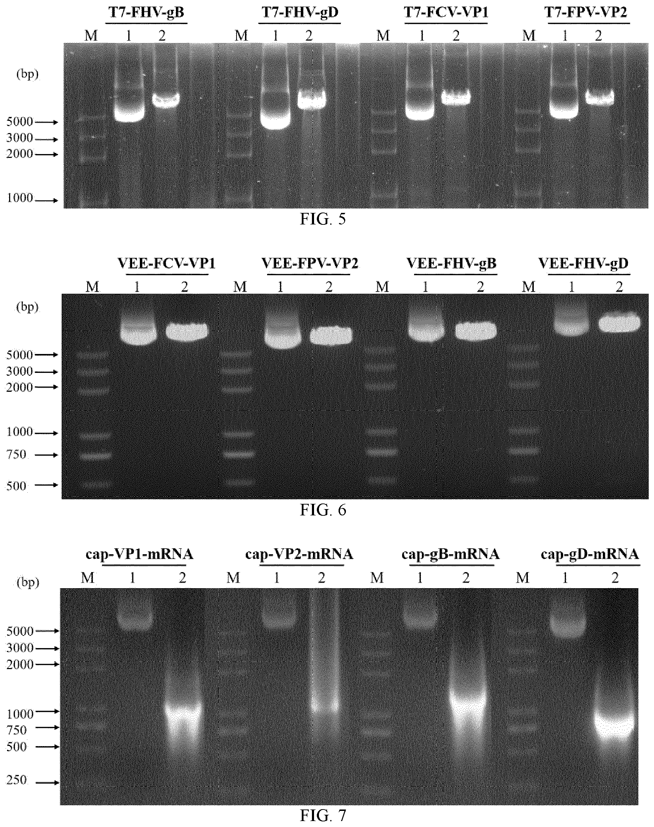

As shown in FIG. 5, the size of the agarose gel electrophoresis band of the synthetic plasmids for the mRNAs was smaller than that of the linearized DNA fragment through restriction digestion, confirming the completion of linearization of T7-FHV-gB, T7-FHV-gD, T7-FCV-VP1, and T7-FPV-VP2 plasmids;

As shown in FIG. 6, the size of the agarose gel electrophoresis band of the synthetic plasmids for the replicating mRNAs is smaller than that of the linearized DNA fragment through restriction digestion, confirming the completion of linearization of VEE-FHV-gB, VEE-FHV-gD, VEE-FCV-VP1, and VEE-FPV-VP2 plasmids.

(2) Using the linearized DNA from the step (1) as a template, the mRNAs and the replicating mRNAs expressing the antigenic proteins were obtained through in vitro transcription.

Using the T7 promoters upstream of the target genes in the synthetic plasmids for the mRNAs, the mRNAs expressing the antigenic proteins of the triple mRNA vaccine for feline rhinotracheitis, feline caliciviral disease, and feline panleukopenia were prepared and expressed via in vitro transcription reactions.
a) First, 1-2 µg of the linearized plasmids for the mRNAs or the linearized plasmids for the replicating mRNAs were taken and used for mRNA synthesis with T7 RNA polymerase at 37°C. The in vitro transcription reaction system was prepared as follows:

| | |
|---|---|
| 10× reaction buffer | 2 µL, |
| ATP/UTP/CTP/GTP (100 mM) | 1.5 µL each, |
| T7 polymerase | 1 µL, |
| Linear DNA template | 1-2 µg, |
| Nuclease-free water | adjust the volume to 20 µL, |

b) The in vitro transcription system was incubated at 37°C for 4 h. Subsequently, 1 µL of DNase was added, and the mixture was incubated at 37°C for 15 min to digest the DNA template; and
c) gB-mRNA (sequence as shown in SEQ ID NO.5), gD-mRNA (sequence as shown in SEQ ID NO.6), VP1-mRNA (sequence as shown in SEQ ID NO.7), and VP2-mRNA (sequence as shown in SEQ ID NO.8) (or VEE-gB-mRNA (sequence as shown in SEQ ID NO.6), VEE-gD-mRNA (sequence as shown in SEQ ID NO.10), VEE-VP1-mRNA (sequence as shown in SEQ ID NO.11), and VEE-VP2-mRNA (sequence as shown in SEQ ID NO. 12)) were extracted via the Trizol method or affinity chromatography. 1 µL was taken and diluted 10-fold to determine the concentrations of the mRNAs, and the mRNAs were stored at -80°C.

### Example 3 Preparation of Cap mRNAs via a Capping Modification Reaction

Using a vaccinia virus capping enzyme and related components, a 7-methylguanosine cap structure was added to the 5' end of mRNAs, making the mRNAs more stable and conducive to transport and translation.
a) The in vitro transcribed gB-mRNA, gD-mRNA, VP1-mRNA, and VP2-mRNA (or VEE-gB-mRNA, VEE-gD-mRNA, VEE-VP1-mRNA, and VEE-VP2-mRNA) were subjected to a capping modification reaction at 37°C using the vaccinia virus capping enzyme and Cap 2'-O-methyltransferase. The capping modification reaction system was prepared as follows:

| | |
|---|---|
| mRNA | 20 µL, |
| 10× capping buffer | 3 µL, |
| GTP (10 mM) | 3 µL, |
| SAM (20 mM) | 0.75 µL, |
| RNase inhibitor | 0.75 µL, |
| Cap 2'-O-methyltransferase (100 U/µL) | 1.2 µL, |
| Vaccinia virus capping enzyme (10 U/µL) | 1.2 µL |

b) The capping modification reaction system was incubated at 37°C for 60 min;
c) The cap-modified mRNAs expressing the antigenic proteins were extracted by the Trizol method or affinity chromatography. 1 µL was taken and diluted 10-fold to determine the concentrations of the mRNAs, and the mRNAs were stored at -80°C; and
d) The cap-modified mRNAs expressing the antigenic proteins were verified through agarose gel electrophoresis, including non-replicating mRNAs: cap-gB-mRNA, cap-gD-mRNA, cap-VP1-mRNA, and cap-VP2-mRNA, as well as replicating mRNAs: cap-VEE-gB-mRNA, cap-VEE-gD-mRNA, cap-VEE-VP1-mRNA, and cap-VEE-VP2-mRNA.

As shown in FIG. 7, agarose gel electrophoresis showed the band sizes of mRNAs. The synthetic plasmids for the mRNAs expressing the antigenic proteins were used as controls, confirming the effective synthesis of the mRNA for the feline herpesvirus gB protein (cap-gB-mRNA), the mRNA for the feline herpesvirus gD protein (cap-gD-mRNA), the mRNA for the feline calicivirus VP1 protein (cap-VP1-mRNA), and the mRNA for the feline parvovirus VP2 protein (cap-VP2-mRNA).

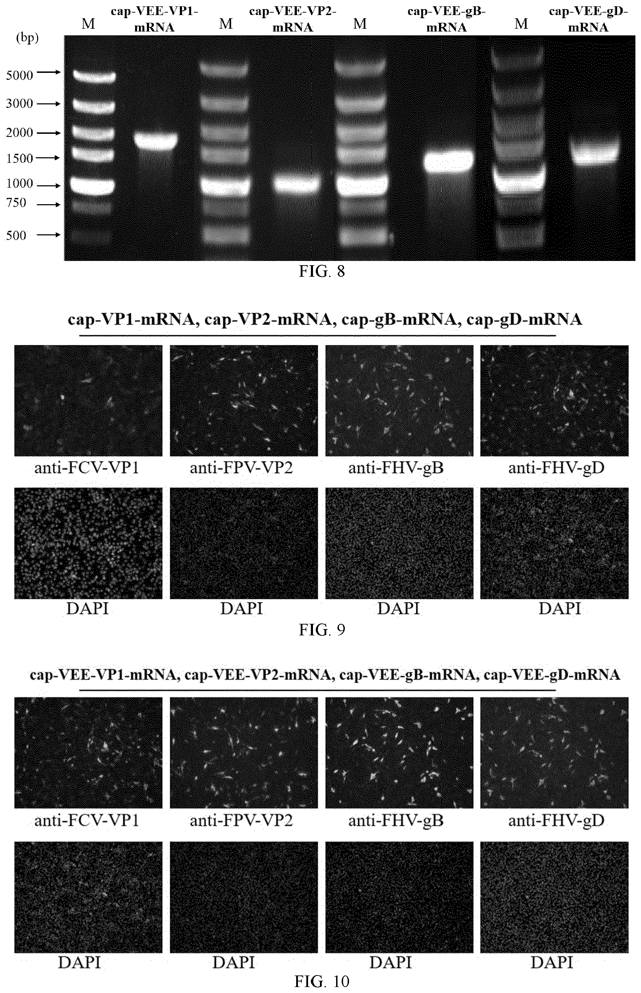

As shown in FIG. 8, agarose gel electrophoresis showed the band sizes of mRNAs, confirming the effective synthesis of the replicating mRNA for the feline herpesvirus gB protein (cap-VEE-gB-mRNA), the replicating mRNA for the feline herpesvirus gD protein (cap-VEE-gD-mRNA), the replicating mRNA for the feline calicivirus VP1 protein (cap-VEE-VP1-mRNA), and the replicating mRNA for the feline parvovirus VP2 protein (cap-VEE-VP2-mRNA).

### Example 4 Immunofluorescence Assay for Detecting Expression of Feline Herpesvirus gB, Feline Herpesvirus gD, Feline Calicivirus VP1, and Feline Parvovirus VP2 Proteins after Transfection of mRNAs Expressing Antigenic Proteins

Transfection of the mRNAs expressing the antigenic proteins into cells enables expression of the feline herpesvirus gB protein, the feline herpesvirus gD protein, the feline calicivirus VP1 protein, and the feline parvovirus VP2 protein. Effective translation of mRNAs expressing antigenic proteins was detected by using specific antibodies against corresponding antigenic proteins, which was performed according to the following steps:
a) BHK-21 adherent cells were seeded in 48-well plates and cultured at 37°C in a 5% CO₂ incubator. When cell confluency reached about 70%, cap-gB-mRNA, cap-gD-mRNA, cap-VP1-mRNA, and cap-VP2-mRNA (or cap-VEE-gB-mRNA, cap-VEE-gD-mRNA, cap-VEE-VP1-mRNA, and cap-VEE-VP2-mRNA) were obtained for transfection;
b) A sterile EP tube was taken, and 300 µL of Opti-MEM medium and 4 µL of DMRIE-C transfection reagent were added, vortexed to mix evenly, and incubated at room temperature for 30 min. Then, 2 µg of cap-gB-mRNA, cap-gD-mRNA, cap-VP1-mRNA, and cap-VP2-mRNA (or cap-VEE-gB-mRNA, cap-VEE-gD-mRNA, cap-VEE-VP1-mRNA, and cap-VEE-VP2-mRNA) were separately added, gently tapped to mix evenly, and incubated for 10 min; and
c) The cells were washed once with Opti-MEM medium, and the pre-incubated mixture was added. After 24 h, specific rabbit polyclonal antibodies against the feline herpesvirus gB protein, the feline herpesvirus gD protein, the feline calicivirus VP1 protein, and the feline parvovirus VP2 protein were used for indirect immunofluorescence to detect gB protein expression.

The specific rabbit polyclonal antibody against the gB protein was derived from antibody serum prepared by immunizing New Zealand white rabbits with synthetic peptides from the gB protein polypeptide segments TPPKPTTDPTDMSDC and GKRRRGSRWQGHSGC conjugated. The specific rabbit polyclonal antibody against the gD protein was derived from antibody serum prepared by immunizing New Zealand white rabbits with synthetic peptides from the gD protein polypeptide segments KRSNDSRGESSGPNC and CDDDVPTAPPKGMNNQSV conjugated. The specific rabbit polyclonal antibody against the VP1 protein was derived from antibody serum prepared by immunizing New Zealand white rabbits with synthetic peptides from the VP1 protein polypeptide segments STLPETGARGGNHPC and TATLDGDNNNKINPC conjugated. The specific rabbit polyclonal antibody against the VP2 protein was derived from antibody serum prepared by immunizing New Zealand white rabbits with synthetic peptides from the VP2 protein polypeptide segments CQPDGGQPAVRNERA and QTDENQAADGDPRYC conjugated.

As shown in FIG. 9, obvious green fluorescent signals were observed in BHK-21 cells co-transfected with cap-gB-mRNA, cap-gD-mRNA, cap-VP1-mRNA, and cap-VP2-mRNA under a fluorescence microscope, indicating that the mRNAs can effectively express the feline herpesvirus gB protein, the feline herpesvirus gD protein, the feline calicivirus VP1 protein, and the feline parvovirus VP2 protein.

As shown in FIG. 10, obvious green fluorescent signals were observed in BHK-21 cells transfected with cap-VEE-gB-mRNA, cap-VEE-gD-mRNA, cap-VEE-VP1-mRNA, and cap-VEE-VP2-mRNA under a fluorescence microscope, indicating that the replicating mRNAs can effectively express the feline herpesvirus gB protein, the feline herpesvirus gD protein, the feline calicivirus VP1 protein, and the feline parvovirus VP2 protein with higher expression levels.

### Example 5 Preparation of Lipid Nanoparticle mRNA Vaccine LNP-gB/gD/VP1/VP2-mRNA and Lipid Nanoparticle Replicating mRNA Vaccine LNP-VEE-gB/gD/VP1/VP2-mRNA

mRNA, a negatively charged biomacromolecule, cannot pass through the negatively charged cell membrane via passive transport. Lipid nanoparticles (LNPs) can be used for RNA delivery and serve as an effective drug delivery approach for mRNA vaccines.

The preparation of lipid nanoparticle mRNA vaccine LNP-gB/gD/VP1/VP2-mRNA or lipid nanoparticle replicating mRNA vaccine LNP-VEE-gB/gD/VP1/VP2-mRNA was performed according to the following steps:
a) SM-102, DSPC, DMG-PEG2000, and cholesterol were dissolved in 100 µL of anhydrous ethanol according to a formulation with a molar ratio of 50:10:38.5:1.5 and a total mass of 480 µg;
b) Under vortexing, the ethanol solution was rapidly injected into 300 µL of 20 mM sodium acetate buffer containing 2 µg each of cap-gB-mRNA and cap-gD-mRNA, and 3 µg each of cap-VP1-mRNA and cap-VP2-mRNA (or 2 µg each of cap-VEE-gB-mRNA and cap-VEE-gD-mRNA, and 3 µg each of cap-VEE-VP1-mRNA and cap-VEE-VP2-mRNA) prepared in Example 3. The mixture was vigorously stirred for 20 s, and then allowed to stand for 10 min to obtain nanoparticles; and
c) The prepared ethanol-sodium acetate mixed solution containing the nanoparticles was dialyzed against 10 mM PBS for 2-4 h to remove ethanol, and subjected to ultrafiltration concentration to obtain the final product LNP-gB+gD+VP1+VP2-mRNA or replicating LNP-VEE-gB+gD+VP1+VP2-mRNA.

### Example 6 Immunization Experiment of the Triple mRNA Vaccine for Feline Rhinotracheitis, Feline Caliciviral Disease, and Feline Panleukopenia

The immunological efficacy of triple mRNA vaccines [LNP-gB+gD+VP1+VP2-mRNA and replicating LNP-VEE-gB+gD+VP1+VP2-mRNA] was evaluated following the procedures below:
a) Forty-four 6-week-old female BALB/c mice were randomly divided into 11 groups: negative control (Mock), non-replicating triple mRNA vaccine group (T7-triple, LNP-gB+gD+VP1+VP2-mRNA vaccine), replicating triple mRNA vaccine group (VEE-triple, replicating LNP-VEE-gB+gD+VP1+VP2-mRNA vaccine), and non-replicating monovalent mRNA vaccine groups (T7-gB group, T7-gD group, T7-VP1 group, T7-VP2 group) and replicating monovalent mRNA vaccine groups (VEE-gB group, VEE-gD group, VEE-VP1 group, VEE-VP2 group). BALB/c mice were intramuscularly injected with the LNP-gB+gD+VP1+VP2-mRNA vaccine (or replicating LNP-VEE-gB+gD+VP1+VP2-mRNA vaccine), or monovalent LNP-gB/gD/VP1/VP2-mRNA vaccine (or monovalent replicating LNP-VEE-gB/gD/VP1/VP2-mRNA vaccine), or mRNA-free LNPs on day 0 and day 30;
b) On day 7 after the second immunization, blood was collected via the orbital route, and the collected serum samples were harvested; and
c) The specific antibody levels against feline herpesvirus, feline calicivirus, and feline parvovirus in serum after mRNA vaccine immunization were determined by double-antibody one-step sandwich enzyme-linked immunosorbent assay: microplate wells pre-coated with feline herpesvirus, feline calicivirus, or feline parvovirus antigens were sequentially added with the serum samples and HRP-labeled detection antibodies, followed by incubation and thorough washing. Substrate TMB was used for color development, and was converted to a final yellow color under the action of 1 M sulfuric acid. The absorbance (OD value) was measured at 450 nm using a microplate reader.

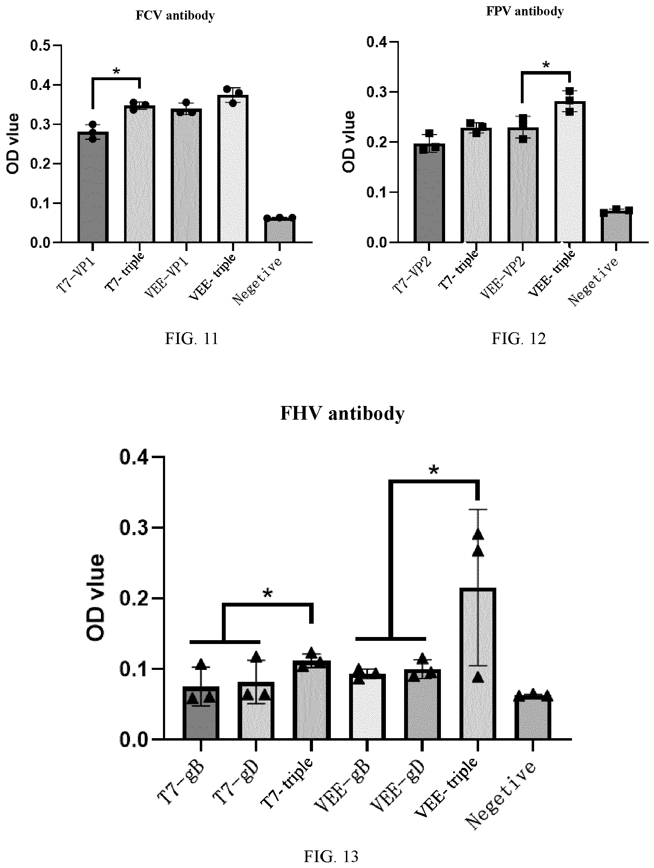

As shown in FIG. 11, the specific antibody level against feline calicivirus (FCV) in the serum samples was determined. Animals immunized with the monovalent mRNA vaccine (T7-VP1), replicating monovalent mRNA vaccine (VEE-VP1), triple mRNA vaccine (T7-triple), or replicating triple mRNA vaccine (VEE-triple) all generated FCV-specific antibodies at different levels. The antibody level in the T7-triple group was significantly higher than that in the monovalent T7-VP1 group, and the antibody level in the replicating VEE-triple group was slightly higher than that in the monovalent VEE-VP1 group. The data indicate that the triple mRNA vaccine for feline rhinotracheitis, feline caliciviral disease, and feline panleukopenia can effectively activate humoral immunity to produce feline calicivirus-specific antibodies, and the non-replicating triple mRNA vaccine elicits a higher antibody level than the monovalent mRNA vaccine.

As shown in FIG. 12, the specific antibody level against feline parvovirus (FPV) in the serum samples was determined. Animals immunized with the monovalent mRNA vaccine (T7-VP2), replicating monovalent mRNA vaccine (VEE-VP2), triple mRNA vaccine (T7-triple), or replicating triple mRNA vaccine (VEE-triple) all generated FPV-specific antibodies at different levels. The antibody level in the T7-triple group was slightly higher than that in the monovalent T7-VP1 group, and the antibody level in the replicating VEE-triple group was significantly higher than that in the monovalent VEE-VP1 group. The data indicate that the triple mRNA vaccine for feline rhinotracheitis, feline caliciviral disease, and feline panleukopenia can effectively activate humoral immunity to produce feline parvovirus-specific antibodies, and the replicating triple mRNA vaccine elicits a higher antibody level than the monovalent mRNA vaccine.

As shown in FIG. 13, the specific antibody level against feline herpesvirus (FHV) in the serum samples was determined. Animals immunized with the monovalent mRNA vaccine (T7-gB, T7-gD), replicating monovalent mRNA vaccine (VEE-gB, VEE-gD), triple mRNA vaccine (T7-triple), or replicating triple mRNA vaccine (VEE-triple) all generated FHV-specific antibodies at different levels. The antibody level in the T7-triple group was significantly higher than those in the monovalent T7-gB and T7-gD groups, and the antibody level in the replicating VEE-triple group was significantly higher than those in the monovalent VEE-gB and VEE-gD groups, with the highest FHV antibody level observed in the replicating VEE-triple group. The data indicate that the triple mRNA vaccine for feline rhinotracheitis, feline caliciviral disease, and feline panleukopenia can effectively activate humoral immunity to produce feline herpesvirus-specific antibodies, and elicits a higher antibody level than the monovalent mRNA vaccine.

## Claims

1. A triple mRNA vaccine for preventing feline rhinotracheitis, feline caliciviral disease, and feline panleukopenia, comprising an mRNA expressing an antigenic protein, wherein the mRNA expressing the antigenic protein comprises an mRNA expressing a feline herpesvirus gB protein, an mRNA expressing a feline herpesvirus gD protein, an mRNA expressing a feline calicivirus VP1 protein, and an mRNA expressing a feline parvovirus VP2 protein.

2. The triple mRNA vaccine according to claim 1, wherein a gene encoding the feline herpesvirus gB protein has a deletion of a nucleotide sequence of positions 2440-2847 at a 3' end, a gene encoding the feline herpesvirus gD protein has a deletion of a nucleotide sequence of positions 988-1125 at a 3' end, and a gene encoding the feline calicivirus VP1 protein has a deletion of a nucleotide sequence of positions 4-372 at a 5' end.

3. The triple mRNA vaccine according to claim 2, wherein the gene encoding the feline herpesvirus gB protein is a codon-optimized gene with a nucleotide sequence as shown in SEQ ID NO.1; the gene encoding the feline herpesvirus gD protein is a codon-optimized gene with a nucleotide sequence as shown in SEQ ID NO.2; the gene encoding the feline calicivirus VP1 protein is a codon-optimized gene with a nucleotide sequence as shown in SEQ ID NO.3; and a gene encoding the feline parvovirus VP2 protein is a codon-optimized gene with a nucleotide sequence as shown in SEQ ID NO.4.

4. The triple mRNA vaccine according to claim 1, wherein the mRNA sequence expressing the feline herpesvirus gB protein is as shown in SEQ ID NO.5 or SEQ ID NO.9, the mRNA sequence expressing the feline herpesvirus gD protein is as shown in SEQ ID NO.6 or SEQ ID NO.10, the mRNA sequence expressing the feline calicivirus VP1 protein is as shown in SEQ ID NO.7 or SEQ ID NO.11, and the mRNA sequence expressing the feline parvovirus VP2 protein is as shown in SEQ ID NO.8 or SEQ ID NO.12.

5. The triple mRNA vaccine according to claim 1, wherein a mass ratio of the mRNA expressing the feline herpesvirus gB protein, the mRNA expressing the feline herpesvirus gD protein, the mRNA expressing the feline calicivirus VP1 protein, and the mRNA expressing the feline parvovirus VP2 protein is 2:2:3:3.

6. The triple mRNA vaccine according to claim 1, further comprising an adjuvant, wherein the adjuvant comprises a lipid nanoparticle adjuvant, an oil-in-water adjuvant, a polymer-and-water-based adjuvant, a water-in-oil adjuvant, an aluminum hydroxide adjuvant, or a vitamin E adjuvant.

7. The triple mRNA vaccine according to claim 6, wherein the adjuvant is a cationic lipid, distearoylphosphatidylcholine, a polyethylene glycol lipid, and cholesterol at a molar ratio of 50:10:38.5:1.5.

8. The triple mRNA vaccine according to claim 1, wherein the triple mRNA vaccine is in a dosage form for intramuscular, intradermal, or subcutaneous administration.

9. A method for preparing the triple mRNA vaccine according to any one of claims 1-8, comprising the following steps:
(1) constructing a synthetic plasmid for an mRNA expressing an antigenic protein, and performing linearization treatment with a restriction endonuclease to obtain a linearized synthetic plasmid for the mRNA expressing the antigenic protein;
(2) performing an in vitro transcription reaction on the linearized synthetic plasmid for the mRNA expressing the antigenic protein using a T7 promoter sequence, and purifying to obtain the mRNA expressing the antigenic protein;
(3) performing a Cap capping modification reaction on the mRNA expressing the antigenic protein obtained in the step (2) by an enzymatic method using a vaccinia virus capping enzyme and 2'-O-methyltransferase, and purifying to obtain a cap-modified mRNA expressing the antigenic protein; and
(4) mixing the cap-modified mRNA expressing the antigenic protein obtained in the step (3) with a cationic lipid, distearoylphosphatidylcholine, cholesterol, and a polyethylene glycol lipid to obtain the triple mRNA vaccine.

10. A triple mRNA vaccine according to any one of claims 1-8 for use in simultaneously preventing feline rhinotracheitis, feline caliciviral disease, and feline panleukopenia.

## Patentansprüche

1. Ein dreifacher mRNA-Impfstoff zur Vorbeugung gegen die feline Rhinotracheitis, die feline Calicivirus-Erkrankung und die feline Panleukopenie, umfassend eine mRNA, die ein antigenes Protein exprimiert, wobei die mRNA, die das antigene Protein exprimiert eine mRNA, die ein gB-Protein des felinen Herpesvirus exprimiert, eine mRNA, die ein gD-Protein des felinen Herpesvirus exprimiert, eine mRNA, die ein VP1-Protein des felinen Calicivirus exprimiert, und eine mRNA, die ein VP2-Protein des felinen Parvovirus exprimiert, umfasst.

2. Der dreifache mRNA-Impfstoff nach Anspruch 1, wobei ein Gen, das für das gB-Protein des felinen Herpesvirus kodiert, eine Deletion einer Nukleotidsequenz an den Positionen 2440-2847 am 3'-Ende hat, ein Gen, das für das gD-Protein des felinen Herpesvirus kodiert, eine Deletion einer Nukleotidsequenz an den Positionen 988-1125 am 3'-Ende hat, und ein Gen, das für das VP1-Protein des felinen Calicivirus kodiert, eine Deletion einer Nukleotidsequenz an den Positionen 4-372 am 5'-Ende hat.

3. Der dreifache mRNA-Impfstoff nach Anspruch 2, wobei das Gen, das für das gB-Protein des felinen Herpesvirus kodiert, ein codonoptimiertes Gen mit einer Nukleotidsequenz wie in SEQ ID NO. 1 gezeigt ist; das Gen, das für das gD-Protein des felinen Herpesvirus kodiert, ein codonoptimiertes Gen mit einer Nukleotidsequenz wie in SEQ ID NO. 2 gezeigt ist; das Gen, das für das VP1-Protein des felinen Calicivirus kodiert, ein codonoptimiertes Gen mit einer Nukleotidsequenz wie in SEQ ID NO. 3 gezeigt ist; und ein Gen, das für das VP2-Protein des felinen Parvovirus kodiert, ein codonoptimiertes Gen mit einer Nukleotidsequenz wie in SEQ ID NO. 4 gezeigt ist.

4. Der dreifache mRNA-Impfstoff nach Anspruch 1, wobei die mRNA-Sequenz, die das gB-Protein des felinen Herpesvirus exprimiert, der in SEQ ID NO. 5 oder SEQ ID NO. 9 gezeigten entspricht, die mRNA-Sequenz, die das gD-Protein des felinen Herpesvirus exprimiert, der in SEQ ID NO. 6 oder SEQ ID NO. 10 gezeigten entspricht, die mRNA-Sequenz, die das VP1-Protein des felinen Calicivirus exprimiert, der in SEQ ID NO. 7 oder SEQ ID NO. 11 gezeigten entspricht, und die mRNA-Sequenz, die das VP2-Protein des felinen Parvovirus exprimiert, der in SEQ ID NO. 8 oder SEQ ID NO. 12 gezeigten Sequenz entspricht.

5. Der dreifache mRNA-Impfstoff nach Anspruch 1, wobei ein Massenverhältnis der mRNA, die das gB-Protein des felinen Herpesvirus exprimiert, der mRNA, die das gD-Protein des felinen Herpesvirus exprimiert, der mRNA, die das VP1-Protein des felinen Calicivirus exprimiert, und der mRNA, die das VP2-Protein des felinen Parvovirus exprimiert, 2∶2∶3∶3 beträgt.

6. Der dreifache mRNA-Impfstoff nach Anspruch 1, ferner umfassend ein Adjuvans, wobei das Adjuvans ein Lipid-Nanopartikel-Adjuvans, ein Öl-in-Wasser-Adjuvans, ein Adjuvans auf Basis von Polymer und Wasser, ein Wasser-in-Öl-Adjuvans, ein Aluminiumhydroxid-Adjuvans oder ein Vitamin-E-Adjuvans umfasst.

7. Der dreifache mRNA-Impfstoff nach Anspruch 6, wobei das Adjuvans aus einem kationischen Lipid, Distearoylphosphatidylcholin, einem Polyethylenglykol-Lipid und Cholesterin in einem Molverhältnis von 50∶10∶38,5∶1,5 besteht.

8. Der dreifache mRNA-Impfstoff nach Anspruch 1, wobei der dreifache mRNA-Impfstoff in einer Arzneiform zur intramuskulären, intradermalen oder subkutanen Verabreichung vorliegt.

9. Ein Verfahren zur Herstellung des dreifachen mRNA-Impfstoffs nach einem der Ansprüche 1 bis 8, umfassend die folgenden Schritte:
(1) Konstruieren eines synthetischen Plasmids für eine mRNA, die ein antigenes Protein exprimiert, und Durchführen einer Linearisierungsbehandlung mit einer Restriktionsendonuklease, um ein linearisiertes synthetisches Plasmid für die mRNA zu erhalten, die das antigene Protein exprimiert;
(2) Durchführen einer in vitro-Transkriptionsreaktion an dem linearisierten synthetischen Plasmid für die mRNA, die das antigene Protein exprimiert, unter Verwendung einer T7-Promotorsequenz und Aufreinigen, um die mRNA zu erhalten, die das antigene Protein exprimiert;
(3) Durchführen einer Capping (Cap)-Modifikationsreaktion an der in Schritt (2) erhaltenen, das antigene Protein exprimierenden mRNA durch ein enzymatisches Verfahren unter Verwendung eines Vacciniavirus-Capping-Enzyms und 2'-O-Methyltransferase und Aufreinigen, um eine Cap-modifizierte, das antigene Protein exprimierende mRNA zu erhalten; und
(4) Mischen der in Schritt (3) erhaltenen, Cap-modifizierten mRNA, die das antigene Protein exprimiert, mit einem kationischen Lipid, Distearoylphosphatidylcholin, Cholesterin und einem Polyethylenglykol-Lipid, um den dreifachen mRNA-Impfstoff zu erhalten.

10. Ein dreifacher mRNA-Impfstoff nach einem der Ansprüche 1 bis 8 zur Verwendung bei der gleichzeitigen Vorbeugung gegen die feline Rhinotracheitis, die feline Calicivirus-Erkrankung und die feline Panleukopenie.

## Revendications

1. Un vaccin à ARNm triple destiné à prévenir la rhinotrachéite féline, la maladie calicivirale féline et la panleucopénie féline, comprenant un ARNm exprimant une protéine antigénique, dans lequel l'ARNm exprimant la protéine antigénique comprend un ARNm exprimant une protéine gB de l'herpèsvirus félin, un ARNm exprimant une protéine gD de l'herpèsvirus félin, un ARNm exprimant une protéine VP1 du calicivirus félin et un ARNm exprimant une protéine VP2 du parvovirus félin.

2. Le vaccin à ARNm triple selon la revendication 1, dans lequel un gène codant pour la protéine gB de l'herpèsvirus félin présente une délétion d'une séquence nucléotidique des positions 2440 à 2847 à l'extrémité 3', un gène codant pour la protéine gD de l'herpèsvirus félin présente une délétion d'une séquence nucléotidique des positions 988 à 1125 à l'extrémité 3', et un gène codant pour la protéine VP1 du calicivirus félin présente une délétion d'une séquence nucléotidique des positions 4 à 372 à l'extrémité 5'.

3. Le vaccin à ARNm triple selon la revendication 2, dans lequel le gène codant pour la protéine gB de l'herpèsvirus félin est un gène à codons optimisés avec une séquence nucléotidique telle qu'indiquée dans la SEQ ID NO. 1 ; le gène codant pour la protéine gD de l'herpèsvirus félin est un gène à codons optimisés avec une séquence nucléotidique telle qu'indiquée dans la SEQ ID NO. 2 ; le gène codant pour la protéine VP1 du calicivirus félin est un gène à codons optimisés avec une séquence nucléotidique telle qu'indiquée dans la SEQ ID NO.3 ; et un gène codant pour la protéine VP2 du parvovirus félin est un gène à codons optimisés avec une séquence nucléotidique telle qu'indiquée dans la SEQ ID NO.4.

4. Le vaccin à ARNm triple selon la revendication 1, dans lequel la séquence d'ARNm exprimant la protéine gB de l'herpèsvirus félin est celle indiquée dans la SEQ ID NO.5 ou la SEQ ID NO.9, la séquence d'ARNm exprimant la protéine gD de l'herpèsvirus félin est celle indiquée dans la SEQ ID NO.6 ou la SEQ ID NO.10, la séquence d'ARNm exprimant la protéine VP1 du calicivirus félin est celle indiquée dans la SEQ ID NO.7 ou la SEQ ID NO.11, et la séquence d'ARNm exprimant la protéine VP2 du parvovirus félin est celle indiquée dans la SEQ ID NO.8 ou la SEQ ID NO.12.

5. Le vaccin à ARNm triple selon la revendication 1, dans lequel un rapport de masse de l'ARNm exprimant la protéine gB de l'herpèsvirus félin, de l'ARNm exprimant la protéine gD de l'herpèsvirus félin, de l'ARNm exprimant la protéine VP1 du calicivirus félin et de l'ARNm exprimant la protéine VP2 du parvovirus félin est de 2∶2∶3∶3.

6. Le vaccin à ARNm triple selon la revendication 1, comprenant en outre un adjuvant, dans lequel l'adjuvant comprend un adjuvant de nanoparticules lipidiques, un adjuvant huile-dans-eau, un adjuvant à base de polymère et d'eau, un adjuvant eau-dans-huile, un adjuvant d'hydroxyde d'aluminium ou un adjuvant de vitamine E.

7. Le vaccin à ARNm triple selon la revendication 6, dans lequel l'adjuvant est un lipide cationique, la distéaroylphosphatidylcholine, un lipide de polyéthylèneglycol et le cholestérol dans un rapport molaire de 50∶10∶38,5∶1,5.

8. Le vaccin à ARNm triple selon la revendication 1, dans lequel le vaccin à ARNm triple est sous une forme galénique pour l'administration intramusculaire, intradermique ou sous-cutanée.

9. Un procédé de préparation du vaccin à ARNm triple selon l'une quelconque des revendications 1 à 8, comprenant les étapes suivantes :
(1) construire un plasmide synthétique pour un ARNm exprimant une protéine antigénique, et effectuer un traitement de linéarisation avec une endonucléase de restriction afin d'obtenir un plasmide synthétique linéarisé pour l'ARNm exprimant la protéine antigénique ;
(2) réaliser une réaction de transcription in vitro sur le plasmide synthétique linéarisé pour l'ARNm exprimant la protéine antigénique à l'aide d'une séquence promotrice T7, puis purifier pour obtenir l'ARNm exprimant la protéine antigénique ;
(3) réaliser une réaction de modification par coiffe (Cap) sur l'ARNm exprimant la protéine antigénique obtenu à l'étape (2) par une méthode enzymatique à l'aide d'une enzyme de coiffage du virus de la vaccine et une 2'-O-méthyltransférase, puis purifier pour obtenir un ARNm modifié par coiffe exprimant la protéine antigénique ; et
(4) mélanger l'ARNm modifié par coiffe exprimant la protéine antigénique obtenu à l'étape (3) avec un lipide cationique, de la distéaroylphosphatidylcholine, du cholestérol et un lipide à base de polyéthylène glycol afin d'obtenir le vaccin à ARNm triple.

10. Un vaccin à ARNm triple selon l'une quelconque des revendications 1 à 8 pour une utilisation dans la prévention simultanée de la rhinotrachéite féline, de la maladie calicivirale féline et de la panleucopénie féline.
